# EUROPEAN PATENT APPLICATION

(11) **EP 2 229 969 A1**
(43) Date of publication of application: **22.09.2010**
(21) Application number: 09356023.3
(22) Date of filing: 16.03.2009
(51) Int. Cl.: A61M 5/32, A61M 5/50

(54) **Injection device**

(71) Applicant: Becton Dickinson France, 38800 Le Pont-de-Claix (FR)
(72) Inventor: Gagnieux, Samuel, 38320 Bresson (FR); Blanc, Jérôme, 38950 Saint Martin le Vinoux (FR); Lanier, Romain, 38000 Grenoble (FR)
(74) Representative: Brédeville, Odile Marie

(57) **Abstract**

The present invention relates to an injection device (100) comprising :
- a container (13), a needle hub (3) movable between an exposed position and a retracted position
- a piston (2) and a plunger rod (4),
- biasing means (8) for moving said needle hub (3) from its exposed position to its retracted position,
- retaining means (12b, 32) for maintaining said needle hub (3) in its exposed position,
- deactivating means (20, 33) for releasing said retaining means upon release of distal pressure exerted on said plunger rod (4),
**characterized in that**
said deactivating means include at least one slope (20) located on the distal face of said piston (2) and a complementary slope (33) located on the proximal face of said needle hub (3), said slope (20) and complementary slope (33) being able to cooperate with each other so as to cause the rotation of said needle hub (3) with respect to said piston (2) and to said container (13) and free the extension of said biasing means (8).

## Description

The invention relates to an injection device provided with a needle and that comprises a mechanism to retract the needle in a controlled manner at the end of the injection.

The distal end of a component or of a device is to be understood as meaning the end furthest from the user's hand and the proximal end is to be understood as meaning the end closest to the user's hand. Likewise, in this application, the "distal direction" is to be understood as meaning the direction of injection, and the "proximal direction" is to be understood as meaning the opposite direction to the direction of injection. The term "inwardly" means towards the device axis, as opposed to the term "outwardly".

An injection device typically comprises a container intended to carry a product to be injected; a needle hub carrying a needle and connected to said container; a plunger rod coupled to a piston, on which a user can press in order to move the piston distally, thereby causing the product to be expelled from the container through a distal opening thereof and said needle, and realizing the injection of said product.

Preferably, in particular to prevent accidental injury after use of the injection device, the needle should not be exposed after the injection. One solution is to make the needle automatically retract inside the injection device container at the end of injection.

Several known injection devices perform this needle retraction by means of a great number of components, which makes the device complicated and expensive to design and to assemble. Moreover, such devices may be bulky because the container of the injection device has to house all the components.

An aspect of the present invention is to provide injection devices provided with a system for retracting the needle at the end of the injection that has a simple design and consists of a limited number of components. A further aspect of the present invention is to provide such an injection device that can operate automatically and passively, without the need for the user to apply an additional effort or to make a specific gesture.

Another difficulty encountered with such injection device is to guaranty the integrity of the product contained in the container before use. Indeed contact between this product and parts of the injection device such as for example the needle, the glue used to attach the needle to the needle hub may have irreversible effects on the product and its integrity. The present invention relates to an injection device comprising at least :
- a container intended to carry a product to be injected, said container comprising an open proximal end and a substantially closed distal end provided with an opening,
- a needle hub carrying a needle and located within said container, said needle hub being movable with respect to said container between at least an exposed position, in which the distal tip of said needle extends beyond the distal end of said container through said opening, and a retracted position in which the distal tip of said needle does not extend beyond the distal end of said container,
- a piston provided in said container, proximally from said needle hub, said piston being movable with respect to said container, the distal movement of said piston being intended to cause the product to be expelled from said container thereby realizing the injection of said product,
- a plunger rod intended to be coupled to said piston at least in the distal direction during the injection upon distal pressure exerted on said plunger rod by a user,
- biasing means coupled to said container and to said needle hub, said biasing means being in one of a stressed or released states and aiming at moving said needle hub from its exposed position to its retracted position when going from its stressed state to its released state,
- retaining means for maintaining said needle hub in its exposed position at least before the end of the injection,
- deactivating means for releasing said retaining means at the end of the injection, thereby enabling said biasing means to move said needle hub from its exposed position to its retracted position upon release of distal pressure exerted on said plunger rod,
**characterized in that**
said deactivating means include at least one slope located on the distal face of said piston and at least a complementary slope located on the proximal face of said needle hub, said slope and complementary slope being able to cooperate with each other so as to cause the rotation of said needle hub with respect to said piston and to said container, thereby allowing said biasing means to go from its stressed state to its released state.

Thanks to this arrangement, the invention makes it possible to provide a simplified injection device. There is no need to provide the injection device with a complex assembly, possibly made of several parts able to move the one with respect to the others. Moreover, the injection device of the invention comprises a mechanism to retract the needle in a controlled manner. In particular, with the injection device of the invention, the user does not have to complete a specific gesture in order to trigger the retraction of the needle. The injection device of the invention is a passively activated one as regards the safety mechanism for retracting the needle at the end of injection. In embodiments, the injection device of the invention can be prefilled.

In an embodiment of the injection device of the invention said retaining means include at least a groove located on said container or on said needle hub, said groove having an horizontal portion and a vertical portion, and at least a lug located respectively on said needle hub or on said container, capable of circulating within said groove, said lug being locked in said horizontal portion when said needle hub is in its exposed position, and said lug being free to move into said vertical portion at the end of injection.

In an embodiment, the injection device further comprises at least a perforable sealing element, located within said container between said needle hub and said piston and realizing the tightness of a chamber comprising said product and delimited by said piston, part of the container walls and said perforable sealing element, a proximal tip of said needle protrudes proximally from said needle hub into said container, and said perforable sealing element is capable of moving from a closed position, in which said perforable sealing element is not in contact with said proximal tip of said needle and is not perforated, to an open position, in which said proximal tip of said needle has perforated said perforable sealing element, upon distal pressure exerted on said plunger rod, thereby allowing the product contained in said chamber to flow through said needle.

In an embodiment of the device of the invention, said perforable sealing element is a deformable membrane attached to the inner wall of the container.

In an alternative embodiment of the device of the invention said perforable element is a deformable membrane attached to the needle hub.

In embodiments of the invention, the proximal end of said container comprises inner radial projections able to cooperate with radial walls of a shaft of said plunger rod; so as to prevent said plunger rod to rotate with respect to said container.

In an embodiment of the injection device of the invention said container includes a sleeve and a distal plug attached at the distal end of said sleeve, said distal plug comprising a distal transversal wall including said opening.

For example, said distal plug may comprise two concentric tubular members, an inner tubular member and an outer tubular member, both extending proximally from said distal transversal wall, and the distal end of said sleeve being received between said two concentric tubular elements, said groove or lug being located on an inner wall of said inner tubular member.

In particular, in such embodiments, said sleeve may be made of glass material. It is therefore possible to store specific medecines which would degrade if stored in a plastic container. Moreover, the manufacture of such a sleeve out of glass material is rendered possible thanks to the general simple tubular shape of said sleeve.

In an embodiment of the injection device said distal plug is made of a material chosen in the group comprising at least plastic, metal, composite material, for example made from two or more constituent materials, thermoplastic and combinations thereof.

In an embodiment of the injection device of the invention said membrane is made of a material chosen in the group comprising at least thermoplastic, rubber, silicon and combinations thereof.

In embodiments of the injection device , said biasing means is a helical spring located between said distal end of said container and said needle hub..

The injection device of the invention will now be further described in reference to the following description and attached drawings in which:
- Figure 1 is a sectional view of the injection device according to the invention before injection,
- Figure 2 is a sectional view of the injection device of figure 1 at the end of the injection just prior to retraction of the needle,
- Figure 3 is a sectional view of the injection device of figure 1 after retraction of the needle,
- Figure 4A is a partial perspective view of the distal plug of the injection device of figure 1,
- Figure 4B is a perspective view of the needle hub of the injection device of figure 1,
- Figure 5 is a cross sectional view along line II of the injection device of figure 1.

Referring now to the drawings, the present invention will now be described in detail.

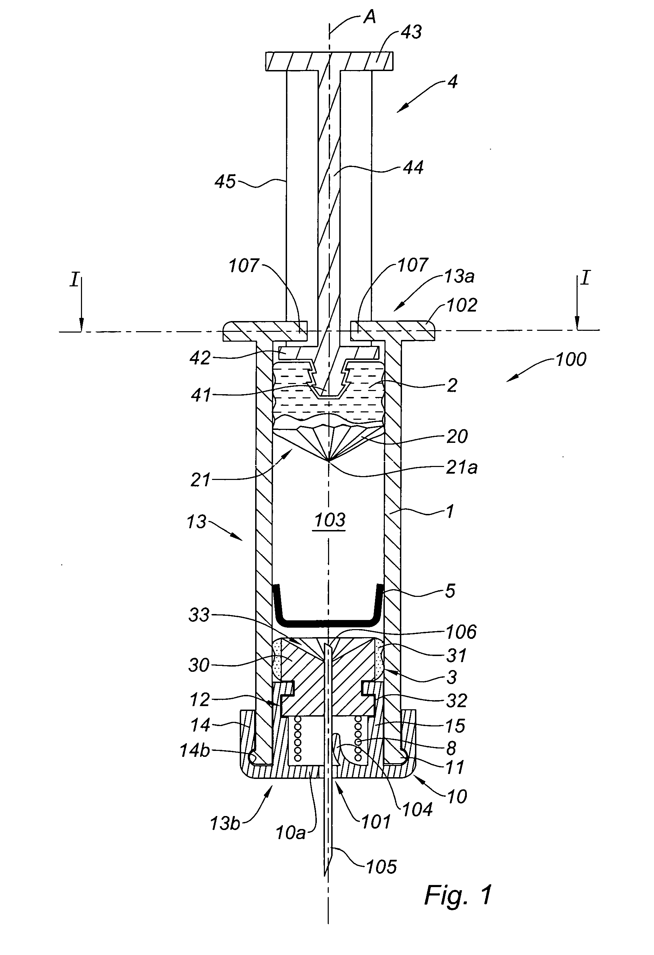

Figure 1 shows a longitudinal cross section of an injection device according to the present invention and generally designated by reference number 100.

The injection device 100 has a longitudinal axis A and comprises a container 13, intended to carry a product to be injected. The container 13 comprises an open proximal end 13a and a substantially closed distal end 13b provided with an opening 101. The container 13 comprises a proximal part formed on the example shown by a sleeve (or barrel) 1 open at both ends and which may be made of glass or plastic.

In particular, when the sleeve 1 is made of glass, its manufacture is very simple since the shape of the sleeve 1 is globally tubular and does not comprise complicated reliefs. The sleeve 1 is provided at its proximal end with outer flanges 102 extending outwardly: such flanges 102 constitute bearing surfaces for the fingers of the user at the time of injection. The sleeve 1 is further provided at its proximal end with inner radial projections 107, extending towards the longitudinal axis A of the injection device 100. On the example shown, the sleeve 1, the inner radial projections 107 and the outer flanges 102 are made of one single element, for example out of glass. In another embodiment not shown, the sleeve 1, the inner radial projections 107 and the outer flanges 102 may be made of several parts attached to each other.

The container 13 further comprises a distal plug 10, mounted at the distal end of the sleeve 1 and closing substantially the distal end 13b of the container 13. The opening 101 is therefore provided on said distal plug 10 on a distal transversal wall 10a of said distal plug 10. The distal transversal wall 10a is provided with an inner flexible cover 104, located in regards to said opening 101 and tending to close said opening 101.

The distal plug 10 is further provided with two concentric tubular members extending in the proximal direction from said distal transversal wall 10a, an outer tubular member 14 and an inner tubular member 15, the distal end of the sleeve 1 being received between said two tubular members (14, 15). The outer tubular member 14 is provided on its inner wall with an inner annular groove 14b, and the sleeve 1 is provided on its outer wall with an external annular rib 11, the distal plug 10 being attached to the sleeve 1 through the external annular rib 11 being engaged in said inner annular groove 14b. In another embodiment not shown, the distal plug and the sleeve are attached to each other by any other suitable means such as for example gluing, friction.

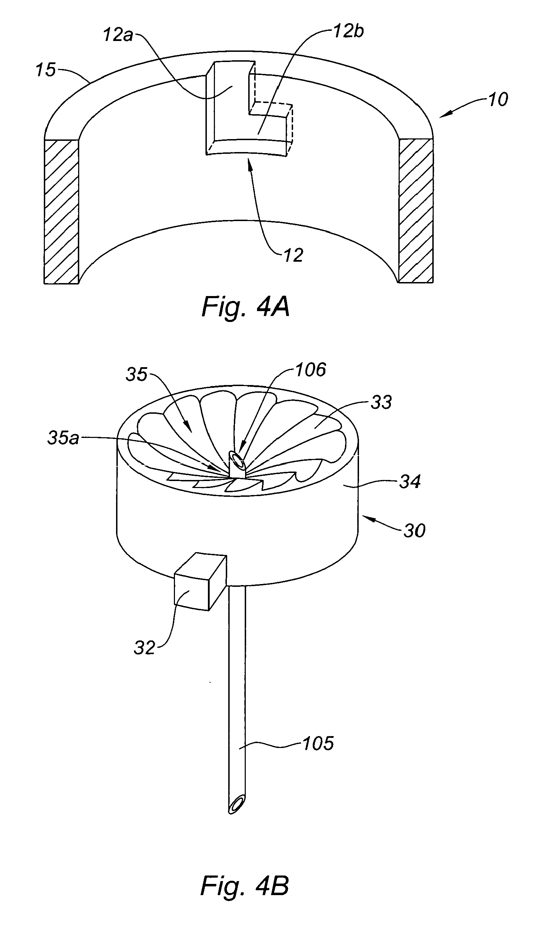

With reference to figure 4A is shown a partial perspective view of the inner tubular member 15 of the distal plug 10. As appears on this figure, the inner wall of the inner tubular member 15 is provided with at least one groove 12 having a L shape. The groove 12 has therefore a vertical portion 12a and a horizontal portion 12b. With reference to figure 1, the inner tubular member 15 of the distal plug 10 has two grooves 12 diametrically opposed.

The distal plug 10 may be made of a material chosen in the group comprising at least plastic, metal, composite material, for example made from two or more constituent materials, and thermoplastic.

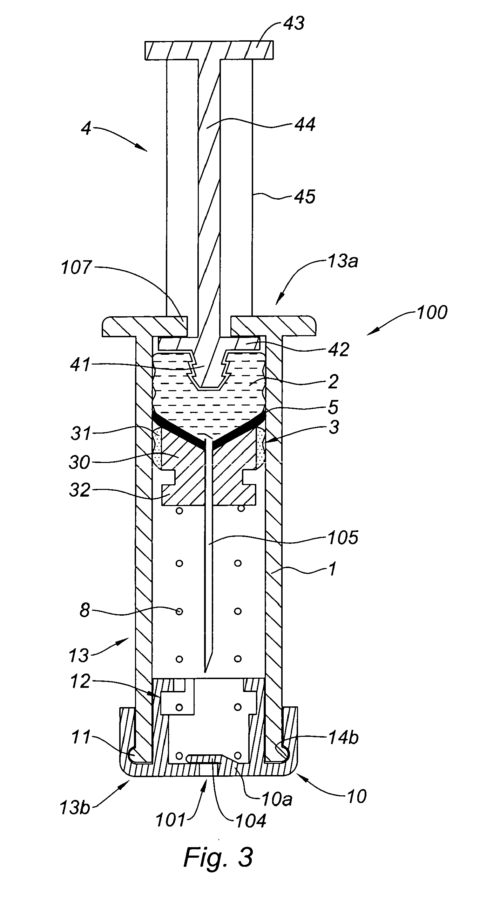

The injection device 100 further comprises a needle hub 3 located within the container 13, at the distal end thereof, and carrying a needle 105. At least part of the needle 105 is embedded in the needle hub 3 by any suitable means such as for example friction, glue. In the before injection position of the injection device 100, as shown on figure 1, the needle 105 extends in the distal direction through the opening 101 and is in contact with the flexible cover 104, forcing said flexible cover 104 in a stressed state so that said flexible cover 104 does not close said opening 101. In this before injection position, the distal tip 105a of the needle 105 extends beyond the distal end 13b of the container 13. As will appear from the description below, the needle hub 3 is movable with respect to the container 1 between the exposed position as shown on figure 1, and a retracted position in which the distal tip 105a of the needle 105 does not extend beyond the distal end 13b of the container 13 (see figure 3), the needle hub 3 and needle 105 being entirely retracted within the container 13 and the flexible cover 104 closing the opening 101.

In the before injection position, the distal end of the container 13 can receive a needle cover (not shown) to protect the needle 105 and prevent needle stick before use of the injection device 100.

On the example shown, the needle hub 3 is made of two coinjected materials defining a needle hub central part 30 and a needle hub outer part 31 receiving at least partially said needle hub central part 30. The central part 30 of the hub 3 is made of a hard material ensuring the attachment of the needle 105. For example, the central part 30 may be made in plastic. The outer part 31 of the hub 3 is made of a softer material ensuring the tightness with the inner wall of the sleeve 1. For example, such soft material may be selected from rubber or thermoplastic. In another embodiment not shown, the needle hub is made of only one part.

With reference to figure 4B, is shown a perspective view of the needle hub central part 30. The needle hub central part 30 has the global shape of a cylinder section 34, the proximal extremity of which has a concave conical shape 35 made of several radial slopes 33. All the radial slopes 33 rejoin at the center 35a of said concave conical shape 35. The proximal end of the needle 105 emerges proximally from said center 35a under the form of a sharpened distal tip 106.

On its outer wall, the cylinder section 34 of the needle hub central part 30 is provided with two diametrically opposed outer lugs 32, one only being visible on figure 4B, each lug 32 being intended to circulate in one L shape groove 12 of the distal plug 10, as will be explained below.

In another embodiment not shown, the needle hub central part can be provided with more lugs and/or lug that are not provided diametrically opposed. In again another embodiment not shown, the lugs can be provided on the distal plug and the grooves on the needle hub.

The injection device 100 further comprises a piston (or stopper) 2 housed in the sleeve 1, proximally spaced from said needle hub 3. The piston 2 is made from a material ensuring the tightness with the inner wall of the sleeve 1, such as elastomeric material. The distal end of the stopper 2 has a convex conical shape 21 made of several radial slopes 20 all rejoining at the center 21 a of the convex conical shape 21. The radial slopes 20 of the piston 2 are complementary to the radial slope 33 of the needle hub central part 30.

In another embodiment not shown, both the concave conical shape of the needle hub central part and the distal end of the stopper are each provided with a single radial slope, the two radial slopes being able to cooperate with each other as described after for the embodiment corresponding to the figures.

The proximal end of the piston 2 is connected with a plunger rod 4 by any suitable means such as for example screwing, bonding. On the example shown, the piston 2 is connected to the plunger rod 4 by means of a screw 41 extending distally from a distal transversal wall 42 of the plunger rod 4, and screwed within said piston 2.

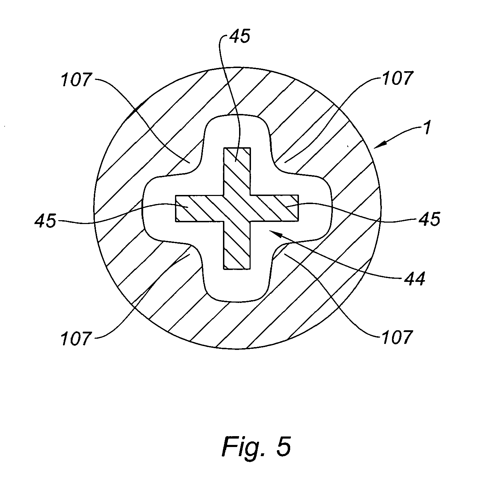

The plunger rod 4 is provided at its proximal end with a proximal transversal wall 43 defining a pushing surface for the user. The distal transversal wall 42 and the proximal transversal wall 43 are linked to each other by means of a shaft 44 having a cross section shape complementary to the shape of the proximal end of the sleeve 1, thereby enabling to prevent the rotation of the plunger rod 4 in regards to the sleeve 1. With reference to figure 5, a cross section view of the proximal end of the sleeve 1 and of the shaft 44 is shown : as appears from this figure, the shaft 44 includes radial walls 45 disposed in-between the inner radial projections 107 of the proximal end of the sleeve 1, so that said shaft 44 is prevented from rotating with respect to said sleeve 1.

Located between the needle hub 3 and the piston 2, the injection device 100 further comprises a flexible concave membrane 5. The membrane 5 is made from a material which may be perforated by the sharpened distal tip 106 of the needle 105. Such a material may be selected from the group comprising at least thermoplastic, rubber and silicon. For example, the membrane 5 is made of Teflon. The membrane 5 is attached to the inner wall of the sleeve 1. Before the injection, as shown on figure 1, the piston 2 is located at the proximal end of the sleeve 1 and defines with the membrane 5 and with the internal wall of the sleeve 1 a chamber (or space) 103 in which the product to be injected is stored. During the storage, the tightness of the product in the injection device 100 is performed by the piston 2 and the membrane 5 with the inner wall of the sleeve 1.

In another embodiment not shown, the injection device is provided without membrane and the storing chamber for the product to be injected is defined by the piston, the internal wall of the container and the needle hub.

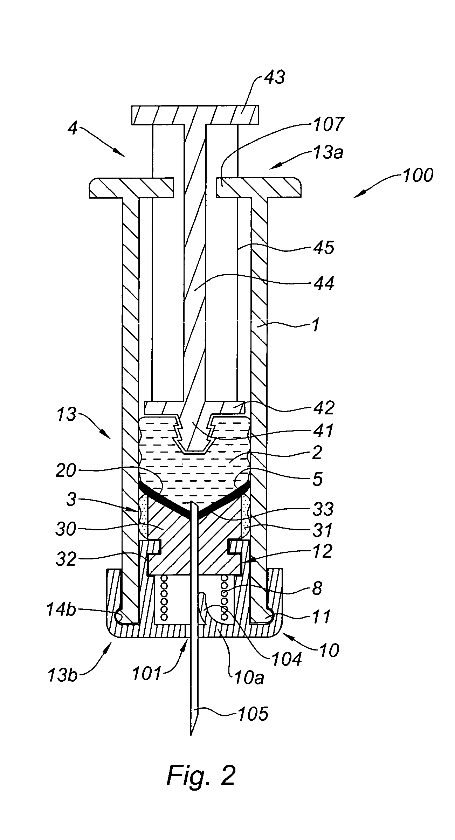

The membrane 5 therefore constitutes a perforable sealing element realizing the tightness of the chamber 103. As will appear from the description below, the membrane or perforable sealing element 5 is capable of moving from a closed position, in which it is not in contact with the proximal tip 106 of the needle 105 and is not perforated (as shown on figure 1), to an open position, in which the proximal tip 106 of the needle 105 has perforated the perforable sealing element or membrane 5, upon distal pressure exerted on the plunger rod 4, thereby allowing the product contained in the chamber 103 to flow through said needle 105 (as shown on figure 2).

As will appear from the description, the piston 2 and the plunger rod 4 are able to move distally from a "before injection" position (see Fig. 1) to an "end of injection" position (see Fig. 2). After injection, the piston 2 and the plunger rod 4 are able to move proximally from the "end of injection" position (see Fig. 2) to a "retracted" position (see Fig. 3).

In the before injection position of figure 1, the needle hub 3 is positioned in order for its slopes 33 to be slightly radially misaligned in regards to the slopes 20 of the piston 2.

The injection device 100 further comprises biasing means, being in one of a stressed or released states and aiming at moving the needle hub from its exposed position to its retracted position when going from its stressed state to its released state, as will appear from the description below. In the example shown on the figures, the biasing means is under the form of a helical spring 8 located between the distal plug 10 and the needle hub 3 and tending to urge them in opposite directions. As appears from figure 1, the proximal end of the helical spring 8 bears on the distal face of the needle hub central part 30 and the distal end of the helical spring 8 bears on the proximal face of the distal transversal wall 10a of the distal plug 10. The helical spring 8 is therefore coupled to the needle hub 3 and to the container 13.

The operation of the injection device 100 of the invention will now be explained with reference to figures 1-3.

The user is provided with the injection device 100 in the position as shown on figure 1. In this position, the helical spring 8 is in a stressed state, a compressed state in the example shown. The lugs 32 of the cylinder section 34 of the needle hub central part 30 are locked in the horizontal portion 12b of the L shaped grooves 12, thereby acting as retaining means of the needle hub 3 in its exposed position. In this position also, the membrane 5 is not perforated.

To use the prefilled injection device 100, the user holds the injection device 100 using the container 13 and the flanges 102, applies it on the injection site (not shown) and applies a distal force on the proximal transversal wall 43 of the plunger rod 4. Under this distal force, the plunger rod 4 moves distally and displaces the piston 2, causing the product contained in the space 103 to deform the membrane 5 toward the needle hub 3 and the sharpened distal tip 106 of the needle 105. The membrane 5 is then pierced by the sharpened distal tip 106, moving therefore to its open position, and allowing the product to be pushed by the piston 2 through the needle 105 and be injected. During the injection step, the lug 32 remains locked in the horizontal portion of the L shape groove 12 and the helical spring 8 is under compression.

As shown on Fig. 2, at the end of the injection, the piston 2 presses the membrane 5 against the needle hub 3. Through the membrane 5, the radial slopes 20 of the piston 2 tend to overlap the complementary radial slopes 33 of the needle hub 3.

Because of the initial radial misalignment between the radial slopes (20, 33) and thus the oblique contact of these radial slopes (20, 33), the distal movement of the piston 2 causes the rotation of the needle hub 3 with respect to the distal plug 10, up to the alignment of the slopes (20, 33). The rotation of the piston 2 is prevented by the specific cross section shape of the plunger rod 4, in other words by the radial walls 45 of the shaft 44 being in abutment against the inner radial projections 107 of the proximal end of the sleeve 1. The rotation of the needle hub 3 causes the circumferential displacement of the lugs 32 along the horizontal portion of the L shape groove 12 up to the vertical portion of the L shape of said groove 12. The radial slopes (20, 33) therefore act as deactivating means of the retaining means (12b, 32). The pressure of the user's thumb on the plunger rod 4 prevents any proximal displacement and the helical spring 8 extension.

Upon release of the pressure applied by the user on the plunger rod 4, the helical spring 8 tends to come back to its natural released state and extends and urges the lugs 32 that are then moved along the vertical portion of the L shape grooves 12 and come out of the grooves 12. Once the lugs 32 are in the vertical portions of the L shape grooves 12, the needle hub 3 is free to move relative to the distal plug 10. The helical spring 8 urges proximally the needle hub 3, the membrane 5, the piston 2, the plunger rod 4 and the needle 105 in the sleeve 1. During this proximal displacement of the needle 105, the flexible cover 104 is free to deflect and finally covers the opening 101 preventing any re-access to the distal end 105a of the needle 105. Any needle stick is thereby prevented.

As shown on Fig. 3, at the end of the proximal displacement of the plunger rod 4, the piston 2, the needle hub 3 and the needle 105, the needle 105 is fully retracted in the sleeve 1. The distal end 105a of the needle therefore does not extend beyond the distal end of the container 13b. During all the retraction, the thumb of the user is on the proximal transversal wall 43 of the plunger rod 4. Therefore, the user is able to control the rate of the retraction of the needle105 inside the injection device 100.

In another embodiment not shown, the membrane is not attached to the sleeve 1 but directly connected to the needle hub 3 and deformable between a closed position in which it is either flat or convex and a open position (under the pressure of the product to be injected pushed by the piston 2) in which it is concave and in contact with the proximal extremity of the needle 105 that pierces it.

The injection device 100 of the invention has advantages over the devices of the prior art in that this injection device 100 is a passively activated one : no additional effort needs to be applied by the user to activate the retraction of the needle at the end of injection. This injection device 100 that contains a limited number of assembled parts is moreover easy to manufacture. The membrane enable to ensure a good isolation of the product to be injected before injection.

Moreover, at least part of the container of the injection device of the invention may be made out of glass material, thereby allowing the storage of specific medicines which would other wise degrade if stored in plastic containers.

## Claims

1. Injection device (100) comprising at least :
- a container (13) intended to carry a product to be injected, said container (13) comprising an open proximal end (13a) and a substantially closed distal end (13b) provided with an opening (101),
- a needle hub (3) carrying a needle (105) and located within said container (13), said needle hub (3) being movable with respect to said container (13) between at least an exposed position, in which the distal tip (105a) of said needle (105) extends beyond the distal end (13b) of said container (13) through said opening (101), and a retracted position in which the distal tip (105a) of said needle (105) does not extend beyond the distal end (13b) of said container (13),
- a piston (2) provided in said container (13), proximally from said needle hub (3), said piston (2) being movable with respect to said container (13), the distal movement of said piston (2) being intended to cause the product to be expelled from said container (13) thereby realizing the injection of said product,
- a plunger rod (4) intended to be coupled to said piston (2) at least in the distal direction during the injection upon distal pressure exerted on said plunger rod (4) by a user,
- biasing means (8) coupled to said container (13) and to said needle hub (3), said biasing means being in one of a stressed or released states and aiming at moving said needle hub (3) from its exposed position to its retracted position when going from its stressed state to its released state,
- retaining means (12b, 32) for maintaining said needle hub (3) in its exposed position at least before the end of the injection,
- deactivating means (20, 33) for releasing said retaining means at the end of the injection, thereby enabling said biasing means to move said needle hub (3) from its exposed position to its retracted position upon release of distal pressure exerted on said plunger rod (4),
**characterized in that**
said deactivating means include at least one slope (20) located on the distal face of said piston (2) and at least a complementary slope (33) located on the proximal face of said needle hub (3), said slope (20) and complementary slope (33) being able to cooperate with each other so as to cause the rotation of said needle hub (3) with respect to said piston (2) and to said container (13), thereby allowing said biasing means (8) to go from its stressed state to its released state.

2. Injection device (100) according to any one of claim 1, wherein said retaining means include at least a groove (12) located on said container (13) or on said needle hub (3), said groove having an horizontal portion (12b) and a vertical portion (12a), and at least a lug (32) located respectively on said needle hub (3) or on said container (13), capable of circulating within said groove (12), said lug (32) being locked in said horizontal portion (12b) when said needle hub (3) is in its exposed position, and said lug (32) being free to move into said vertical portion (12a) at the end of injection.

3. Injection device (100) according to any one of claims 1 or 2, wherein it further comprises at least a perforable sealing element (5), located within said container (13) between said needle hub (3) and said piston (2) and realizing the tightness of a chamber (103) comprising said product and delimited by said piston (2), part of the container (13) walls and said perforable sealing element (5),
a proximal tip (106) of said needle (105) protrudes proximally from said needle hub (3) into said container (13), and
said perforable sealing element (5) is capable of moving from a closed position, in which said perforable sealing element (5) is not in contact with said proximal tip (106) of said needle (105) and is not perforated, to an open position, in which said proximal tip (106) of said needle (105) has perforated said perforable sealing element (5), upon distal pressure exerted on said plunger rod (4), thereby allowing the product contained in said chamber (103) to flow through said needle (105).

4. Injection device (100) according to claim 3, wherein said perforable sealing element is a deformable membrane (5) attached to the inner wall of the container (13).

5. Injection device (100) according to claim 3, wherein said perforable element is a deformable membrane attached to the needle hub (3).

6. Injection device (100) according to any one of claims 1 to 5, wherein the proximal end (13a) of said container (13) comprises inner radial projections (107) able to cooperate with radial walls (45) of a shaft (44) of said plunger rod (4); so as to prevent said plunger rod (4) to rotate with respect to said container (13).

7. Injection device (100) according to any one of claims 1 to 6, wherein said container (13) includes a sleeve (1) and a distal plug (10) attached at the distal end of said sleeve (1), said distal plug (10) comprising a distal transversal wall (10a) including said opening (101).

8. Injection device (100) according to claims 2 and 7, wherein said distal plug (10) comprises two concentric tubular members, an inner tubular member (15) and an outer tubular member (14), both extending proximally from said distal transversal wall (10a), and the distal end of said sleeve (1) being received between said two concentric tubular elements (14, 15), said groove (12) or lug (32) being located on an inner wall of said inner tubular member (15).

9. Injection device (100) according to one of claims 7 or 8, wherein said sleeve (1) is made of glass material.

10. Injection device (100) according to one of claims 7 to 9, wherein said distal plug (10) is made of a material chosen in the group comprising at least plastic, metal, composite material, thermoplastic and combinations thereof.

11. Injection device (100) according to any one of claims 2 to 10, wherein said membrane (5) is made of a material chosen in the group comprising at least thermoplastic, rubber, silicon and combinations thereof.

12. Injection device (100) according to any one of claims 1 to 11, wherein said biasing means is a helical spring (8) located between said distal end of said container (13) and said needle hub (3).
